# FASCICULE DE BREVET EUROPEEN

(11) **EP 2 197 899 B1**
(45) Date de publication et mention de la délivrance du brevet: **03.09.2014**
(21) Numéro de dépôt: 08867746.3
(22) Date de dépôt: 08.10.2008
(51) Int. Cl.: C07K 7/56, A61K 38/00

(54) **PEPTIDES CYCLIQUES COMPRENANT AU MOINS UN RESIDU AZA-BETA-3 AMINOACYCLE ET LEURS UTILISATIONS**
ZYKLISCHE PEPTIDE MIT MINDESTENS EINEM AZA-BETA3-AMINOACYL-REST UND ANWENDUNGEN DAVON
CYCLIC PEPTIDES COMPRISING AT LEAST ONE AZA- BETA3-AMINOACYL RESIDUE AND USES THEREOF

(30) Priorité: 10.10.2007 FR 0707108
(43) Date de publication de la demande: 23.06.2010
(73) Titulaire: Centre National de la Recherche Scientifique, 75794 Paris Cedex 16 (FR); Université de Rennes I, 35065 Rennes Cedex (FR); Baudy Floc'h, Michèle, 35000 Rennes (FR)
(72) Inventeur: LAURENCIN, Mathieu, F-35000 Rennes (FR); ZATYLNY-GAUDIN, Céline, F-14760 Bretteville sur Odon (FR); HENRY, Joël, F- 14400 Bayeux (FR); BAUDY FLOC'H, Michèle, F-35000 Rennes (FR)
(74) Mandataire: Grosset-Fournier, Chantal Catherine
(86) Numéro de dépôt international: PCT/FR2008/001403
(87) Numéro de publication internationale: WO 2009/083663

(56) Documents cités:
- WO-A-2004/111086
- FR-A1- 2 922 211
- FR-A1- 2 925 502
- BUSNEL O ET AL.: "Structure-activity studies of cyclic aza- beta-3-RGD peptide analogs" JOURNAL OF PEPTIDE SCIENCE, vol. 12, no. Suppl. S, septembre 2006 (2006-09), page 169, XP009099748 & 29TH EUROPEAN PEPTIDE SYMPOSIUM; GDANSK, POLAND; SEPTEMBER 03 -08, 2006 ISSN: 1075-2617
- LE GREL PHILIPPE ET AL.: "Aza-beta3-cyclohexapeptides: pseudopeptidic macrocycles with interesting conformational and configurational properties slow pyramidal nitrogen inversion in 24-membered rings!" THE JOURNAL OF ORGANIC CHEMISTRY, vol. 71, no. 15, 21 juillet 2006 (2006-07-21), pages 5638-5645, XP009096823 ISSN: 0022-3263 cité dans la demande
- LENMAN, MORAG M. ET AL.: "Synthesis of fused 1,2,5-triazepine-1,5-diones and some N2- and N3-substituted derivatives: potential conformational mimetics for cis-peptidyl prolinamides" JOURNAL OF THE CHEMICAL SOCIETY, PERKIN TRANSACTIONS 1: ORGANIC AND BIO-ORGANIC CHEMISTRY, CODEN: JCPRB4; ISSN: 0300-922X, DOI: 10.1039/A702107K, no. 16, 1997, pages 2297-2311, XP009099828
- LEGRAND BAPTISTE ET AL.: "Auto-assembling antimicrobial cyclic pseudopeptides including aza-beta(3)-amino acids" JOURNAL OF PEPTIDE SCIENCE, vol. 14, no. 8, Suppl. S, août 2008 (2008-08), page 60, XP9119061 & 30TH EUROPEAN PEPTIDE SYMPOSIUM; HELSINKI, FINLAND; AUGUST 31 SEPTEMBER 05, 2008 ISSN: 1075-2617

### SEQUENCE LISTING

<110> CNRS LAURENCIN, Mathieu ZATYLNY-GAUDIN, Céline HENRY, Joël BAUDY-FLOC'H, Michèle
<120> PEPTIDES CYCLIQUES COMPRENANT AU MOINS UN RESIDU AZA ß3 AMINOACYLE ET LEURS UTILISATIONS
<130> WOB 07 CA CNR AZAB
<150> FR 0707108
   <151> 2007-10-10
<160> 25
<170> PatentIn version 3.5
<210> 1
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1=azaß3K
<220>
   <221> MISC_FEATURE
   <222> (4)..(4)
   <223> Xaa2=azaß3(1)Nal
<220>
   <221> MISC_FEATURE
   <222> (6)..(6)
   <223> Xaa2=azaß3(1)Nal
<400> 1
   Leu Xaa Lys Xaa Leu Xaa
   1               5
<210> 2
   <211> 6
   <212> PRT
   <213> Séquence artificielle
<220>
   <223> Peptide cyclique contenant trois résidus aza-ß3-aminoacyle
<220>
   <221> MISC_FEATURE
   <222> (2) .. (2)
   <223> Xaa1= azaß3K
<220>
   <221> MISC_FEATURE
   <222> (4)..(4)
   <223> Xaa2= azaß3Nal
<220>
   <221> MISC_FEATURE
   <222> (6)..(6)
   <223> Xaa3=azaß3Nal
<400> 2
   Lys Xaa Leu Xaa Leu Xaa
   1               5
<210> 3
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1=azaß3K
<220>
   <221> MISC_FEATURE
   <222> (4)..(4)
   <223> Xaa2=azaß3L
<220>
   <221> MISC_FEATURE
   <222> (6)..(6)
   <223> Xaa2=azaß3L
<400> 3
   Lys Xaa Trp Xaa Trp Xaa
   1               5
<210> 4
   <211> 6
   <212> PRT
   <213> Séquence artificielle
<220>
   <223> peptide cyclique comprenant trois résidus aza-ß3-aminoacyle
<220>
   <221> MISC_FEATURE
   <222> (2)..(2)
   <223> Xaa1= azaß3K
-<220>

   <221> MISC_FEATURE
   <222> (4)..(4)
   -<223> Xaa2=azaß3L
<220>
   <221> MISC_FEATURE
   <222> (6)..(6)
   <223> Xaa3=azaß3L
<400> 4
   Lys Xaa Lys Xaa Trp Xaa
   1               5
<210> 5
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1=azaß3K
<220>
   <221> MISC_FEATURE
   <222> (4)..(4)
   <223> Xaa2=azaß3K
<220>
   <221> MISC_FEATURE
   <222> (6)..(6)
   <223> Xaa3=azaß3L
<400> 5
   Trp Xaa Lys Xaa Trp Xaa
   1               5
<210> 6
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3K
<220>
   <221> MISC_FEATURE
   <222> (6)..(6)
   <223> Xaa3 = azaß3(1)Nal
<400> 6
   Lys Xaa Lys Xaa Trp Xaa
   1               5
<210> 7
   <211> 8
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3(1)Nal)
<220>
   <221> MISC_FEATURE
   <222> (4)..(4)
   <223> Xaa2 = azaß3K
<220>
   <221> MISC_FEATURE
   <222> (6)..(6)
   <223> Xaa3 = azaß3K
<220>
   <221> MISC_FEATURE
   <222> (8)..(8)
   <223> Xaa4 = azaß3K
<400> 7
   Ser Xaa Phe Xaa Thr Xaa Ser Xaa
   1 5
<210> 8
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3R
<220>
   <221> MISC_FEATURE
   <222> (4)..(4)
   <223> Xaa2 = azaß3(1)Nal
<220>
   <221> MISC_FEATURE
   <222> (6)..(6)
   <223> Xaa3 = azalß3(1)Nal
<400> 8
   Leu Xaa Arg Xaa Leu Xaa
   1 5
<210> 9
   <211> 6
   <212> PRT
   <213> Séquence artificielle
<220>
   <223> Peptide cyclique comprenant quatre résidus aza-ß3-aminoacyle
<220>
   <221> MISC_FEATURE
   <222> (2)..(2)
   <223> Xaa1= azaß3K
<220>
   <221> MISC_FEATURE
   <222> (4)..(4)
   <223> Xaa2 = azaß3(1)Nal
<220>
   <221> MISC_FEATURE
   <222> (5)..(5)
   <223> Xaa3= azaß3L
<220>
   <221> MISC_FEATURE
   <222> (6)..(6)
   <223> Xaa4 = azaß3(1)Nal
<400> 9
   Leu Xaa Lys Xaa Xaa Xaa
   1               5
<210> 10
   <211> 6
   <212> PRT
   <213> Séquence artificielle
<220>
   <223> Peptide cyclique comprenant trois résidus aza-ß3-aminocyclique
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3(1)Nal
<220>
   <221> MISC_FEATURE
   <222> (6)..(6)
   <223> Xaa3 = azaß3(1)Nal
<400> 10
   Phe Xaa Lys Xaa Phe Xaa
   1               5
<210> 11
   <211> 5
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3L
<220>
   <221> MISC_FEATURE
   <222> (5)..(5)
   <223> Xaa3 = azaß3(1)Nal
<400> 11
   Leu Xaa Lys Xaa Xaa
   1               5
<210> 12
   <211> 5
   <212> PRT
   <213> Séquence artificielle
<220>
   <223> Peptide cyclique comprenant trois résidus aza-ß3-aminoacyle
<220>
   <221> MISC_FEATURE
   <222> (1)..(1)
   <223> Xaa1 = azaß3K
<220>
   <221> MISC_FEATURE
   <222> (3)..(3)
   <223> Xaa2 = azaß3 (1)Nal
<220>
   <221> MISC_FEATURE
   <222> (5)..(5)
   <223> Xaa3 = azaß3(1)Nal
<400> 12
   Xaa Lys Xaa Leu Xaa
   1               5
<210> 13
   <211> 6
   <212> PRT
   <213> Séquence artificielle
<220>
   <223> Peptide cyclique comprenant trois résidus aza- ß3-aminoacyle
<220>
   <221> MISC_FEATURE
   <222> (2)..(2)
   <223> Xaa1=azaß3K
<220>
   <221> MISC_FEATURE
   <222> (4)..(4)
   <223> Xaa2=azaß3(1)Nal
<220>
   <221> MISC_FEATURE
   <222> (6)..(6)
   <223> Xaa3=azaß3(4F-1)Nal
<400> 13
   Leu Xaa Lys Xaa Leu Xaa
   1               5
<210> 14
   <211> 6
   <212> PRT
   <213> Séquence artificielle
<220>
   <223> Peptide cyclique contenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3Oct
<220>
   <221> MISC_FEATURE
   <222> (6)..(6)
   <223> Xaa3 = azaß3Oct
<400> 14
   Lys Xaa Trp Xaa Trp Xaa
   1               5
<210> 15
   <211> 6
   <212> PRT
   <213> Séquence artificielle
<220>
   <223> Peptide cyclique contenant trois résidus aza-ß3-aminoacyle
<220>
   <221> MISC_FEATURE
   <222> (2)..(2)
   <223> Xaa1=azaß3K
<220>
   <221> MISC_FEATURE
   <222> (3)..(3)
   <223> Xaa2=cha (cyclohexyalanine)
<220>
   <221> MISC_FEATURE
   <222> (4)..(4)
   <223> Xaa3=azaßAmy
<220>
   <221> MISC_FEATURE
   <222> (6)..(6)
   <223> Xaa4=azaß3(1)Nal
<400> 15
   Lys Xaa Xaa Xaa Trp Xaa
   1               5
<210> 16
   <211> 6
   <212> PRT
   <213> Séquence artificielle
-<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (3)..(3)
   <223> Xaa2 = Cha (cyclohexylalanine)
<220>
   <221> MISC_FEATURE
   <222> (4)..(4)
   <223> Xaa3 = azaß3(4)Fpa
<220>
   <221> MISC_FEATURE
   <222> (6)..(6)
   <223> Xaa4 = azaß3Cha
<400> 16
   Arg Xaa Xaa Xaa Trp Xaa
   1               5
<210> 17
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2).. (2)
   <223> Xaa1 = azaß3K
<220>
   <221> MISC_FEATURE
   <222> (4)..(4)
   <223> Xaa2 = azaß3(2)Nal
<220>
   <221> MISC_FEATURE
   <222> (6)..(6)
   <223> Xaa3 = azaß3(2)Nal
<400> 17
   Leu Xaa Lys Xaa Leu Xaa
   1               5
<210> 18
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3(2)Nal
<220>
   <221> MISC_FEATURE
   <222> (6)..(6)
   <223> Xaa3 = azaß3(2)Nal
<400> 18
   Phe Xaa Arg Xaa Phe Xaa
   1               5
<210> 19
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE.
   <222> (4)..(4)
   <223> Xaa2 = azaß3(1)Nal
<220>
   <221> MISC_FEATURE
   <222> (6)..(6)
   <223> Xaa3 = azaß3(4)Fluo-(1)Nal
<400> 19
   Leu Xaa Lys Xaa Leu Xaa
   1               5
<210> 20
   <211> 6
   <212> PRT
   <213> Séquence artificielle
<220>
   <223> Peptide cyclique comprenant trois résidus aza-ß3-aminoacyle
<220>
   <221> MISC_FEATURE
   <222> (2)..(2)
   <223> Xaa1 = azaß3K
<220>
   <221> MISC_FEATURE
   <222> (4)..(4)
   <223> Xaa2 = azaß3Bip
<220>
   <221> MISC_FEATURE
   <222> (6)..(6)
   <223> Xaa3 = azaß3Bip
<400> 20
   Leu Xaa Lys Xaa Leu Xaa
   1               5
<210> 21
   <211> 5
   <212> PRT
   <213> Séquence artificielle
<220>
   <223> Peptide cyclique comprenant trois résidus aza-ß3-aminoacyle
<220>
   <221> MISC_FEATURE
   <222> (1)..(1)
   <223> Xaa1 = azaß3K
<220>
   <221> MISC_FEATURE
   <222> (3)..(3)
   <223> Xaa2 = azaß3Bip
<220>
   <221> MISC_FEATURE
   <222> (5)..(5)
   <223> Xaa3 = azaß3Bip
<400> 21
   Xaa Lys Xaa Leu Xaa
   1               5
<210> 22
   <211> 5
   <212> PRT
   <213> Séquence artificielle
<220>
   <223> Peptide cyclique comprenant trois résidus aza-ß3-aminoacyle
<220>
   <221> MISC_FFATURE
   <222> (2)..(2)
   <223> Xaa1 = azaß3R
<220>
   <221> MISC_FEATURE
   <222> (4)..(4)
   <223> Xaa2 = azaß3(1)Nal
<400> 22
   Leu Xaa Arg Xaa Leu
   1               5
<210> 23
   <211> 5
   <212> PRT
   <213> Séquence artificielle
<220>
   <223> Peptide cyclique comprenant deux résidus aza-ß3-aminoacyle
<220>
   <221> MISC_FEATURE
   <222> (2)..(2)
   <223> Xaa1 = azaß3K
<220>
   <221> MISC_FEATURE
   <222> (5)..(5)
   <223> Xaa2 = azaß3(1)Nal
<400> 23
   Leu Xaa Lys Leu Xaa
   1               5
<210> 24
   <211> 5
   <212> PRT
   <213> Séquence artificielle
<220>
   <223> Peptide cyclique comprenant trois résidus aza-ß3-aminoacyle
<220>
   <221> MISC_FEATURE
   <222> (1)..(1)
   <223> Xaa1 = azaß3K
<220>
   <221> MISC_FEATURE
   <222> (3)..(3)
   <223> Xaa2 = azaß3(2)Nal
<220>
   <221> MISC_FEATURE
   <222> (5)..(5)
   <223> Xaa3 = azaß3(2)Nal
<400> 24
   Xaa Arg Xaa Phe Xaa
   1               5
<210> 25
   <211> 5
   <212> PRT
   <213> Séquence artificielle
<220>
   <223> Peptide cyclique comprenant trois résidus aza-ß3-aminoacyle
<220>
   <221> MISC_FEATURE
   <222> (1)..(1)
   <223> Xaa1 = azaß3K
<220>
   <221> MISC_FEATURE
   <222> (3)..(3)
   <223> Xaa2 = azaß3(2)Nal
<220>
   <221> MISC_FEATURE
   <222> (5)..(5)
   <223> Xaa3 = azaß3(2)Nal
<400> 25
   Xaa Lys Xaa Leu Xaa
   1               5

## Description

La présente invention a pour objet des peptides cycliques comprenant au moins un résidu azaβ³ aminoacyle, ainsi que leurs utilisations dans des compositions pharmaceutiques.

La plupart des médicaments anti-infectieux disponibles appartiennent à la classe des petites molécules chimiques mais de nombreux projets en recherche et développement s'intéressent aujourd'hui à la classe des peptides thérapeutiques.

En effet les propriétés anti-infectieuses de certains peptides sont le sujet de découvertes intéressantes dans le domaine antibactérien, les souches bactériennes devenant de plus en plus résistantes aux antimicrobiens, d'où la nécessité de trouver de nouvelles molécules actives.

Récemment, un groupe a montré qu'il pouvait construire des peptides antibactériens et antifongiques de très petite taille constitués seulement de quatre acides aminés. Un des acides aminés est remplacé par son énantiomère et une chaîne d'acides gras est attachée à la séquence peptidique (C₁₆-KKkK). Dans ce cas, la structure en trois dimensions ne semble pas critique pour l'activité anti-infectieuse du peptide (Makovitzki A., Avrahami D., Shai, Y. PNAS, 2006, 10, 15997). En revanche, la séquence du peptide et la longueur de l'acide gras déterminent le spectre d'activité antimicrobienne.

Une nouvelle famille a également été décrite, il s'agit de peptides cycliques, alternant acides aminés L et D (Fernandez-Lopez S., Kim HS., Choi E., Delgado M. Granja JR., Khazanov A., Kraehenbuehl K., Long G., Weinberger DA, Wilcoxen KM., Ghadiri MR., Nature., 2001, 412, 452 ; Dartois, V., Sanchez-Quesada J., Cabezas E., Chi E., Dubbelde C., Dunn C., Granja J., Gritzen C., Weinberger D., Ghadiri M.R., Parr T.R. Antimicrob. Agents Chemother. 2005, 49, 3302 ; WO 02/090503). Ces peptides synthétiques présentant des propriétés antimicrobiennes s'auto-structurent et sont capables de cibler les membranes microbiennes puis d'y former des brèches conduisant à la destruction des bactéries *in vitro* et *in*-*vivo*.

Cette diversité de structures et de types d'action confèrent aux peptides antimicrobiens un très fort potentiel d'agent anti-infectieux face aux bactéries multi-résistantes. Plusieurs molécules sont d'ailleurs en stade avancé de développement dans le cadre d'infections localisées et les premiers candidats contre les infections systémiques sont actuellement en cours d'évaluation.

Ces peptides antimicrobiens sont l'un des éléments clefs de la défense immunitaire innée des organismes multicellulaires et sont ainsi présents dans le règne animal comme dans le règne végétal. Aussi, les nanostructures à base de peptides offrent de nombreuses possibilités de variation et permettent d'accéder à de nouveaux objets nanoscopiques.

Pour faire face à l'émergence de nouveaux pathogènes et surtout à l'apparition des microorganismes multi-résistants, il est indispensable d'identifier de nouveaux antibiotiques.

Toutefois l'utilisation des peptides est limitée par une rapide élimination en milieu biologique, instabilité métabolique liée à leur dégradation rapide par les peptidases au niveau des liaisons amides. En outre à l'exception des structures purement biologiques, il existe peu de pseudopeptides antimicrobiens biomimétiques possédant une activité et une stabilité supérieures au peptide natif.

Aussi les Inventeurs ont-ils développé la synthèse d'analogues d'acides aminés, les aza-β³-amino acides, qui, une fois incorporés dans les séquences peptidiques, forment des pseudopeptides dont la durée de vie est améliorée

Ces aza-β³-aminoacides ou acides hydrazino acétiques *N*^{α}-substitués (Cheguillaume A., Doubli-Bounoua I., Baudy-Floc'h M., Le Grel P. Synlett, 2000, 3, 331 ; WO 2004/111086) sont des monomères qui peuvent être considérés comme des dérivés aza des β³-aminoacides. Les aza-β³-aminoacides portent leur chaîne latérale sur un atome d'azote à configuration non fixée, modification qui se traduit par une perte de la chiralité du monomère par rapport aux β³-aminoacides. Toutefois grâce à l'inversion plus ou moins rapide de l'azote, ce monomère va pouvoir mimer l'orientation de la chaîne latérale d'un β³-aminoacide de configuration R ou S. D'autre part, l'allongement de la chaîne par un groupement méthylène va apporter une certaine flexibilité au monomère.

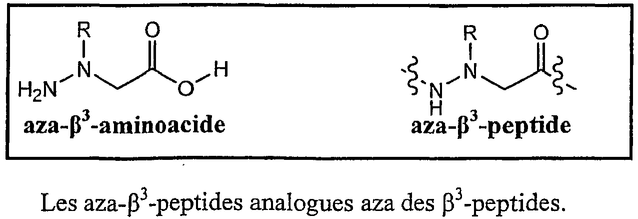

La capacité d'inversion de la configuration de la chaîne latérale au niveau de l'atome d'azote a été validée par la cristallisation d'aza-β³-hexapeptides cycliques. En effet, l'étude RX de ces cristaux montre que les azotes portant les chaînes latérales de ces oligomères alternent configuration R et S tout au long de la séquence. Les aza-β³-cyclohexapeptides existent donc sous la forme de deux invertomères en équilibre semblables au deux formes chaises du cyclohexane (Le Grel P., Salaün A., Potel M., Le Grel B., Lassagne F., J. Org. Chem,. 2006)

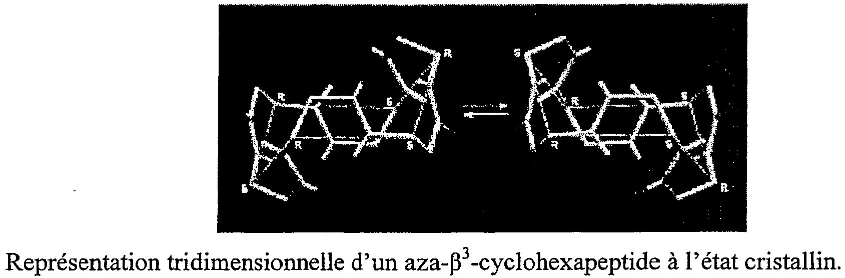

Les aza-β³-aminoacides peuvent donc prendre les deux configurations et sont capables de former des liaisons hydrogène.

Les Inventeurs ont découvert de manière surprenante qu'en faisant alterner des α-aminoacides de configuration fixée et des aza-β³-aminoacides dans des peptides cycliques, on fixe la configuration des azotes, formant ainsi des structures chirales présentant convenablement les chaînes latérales pour former des nanotubes pouvant perforer les membranes des bactéries.

Aussi la présente invention a-telle pour objet des peptides cycliques présentant une alternance aléatoire de résidus L-α-aminoacyles et de résidus aza-β³-aminoacyles répondant à la formule (A) qui suit : 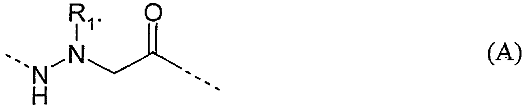 dans laquelle
RI représente une chaîne latérale choisie dans le groupe comprenant les chaînes protéogéniques et non protéogéniques, à condition que deux résidus L-α-aminoacyles soient séparés par au moins un résidu aza-β³-aminoacyle et que le nombre total des résidus L-α-aminoacyles et des résidus aza-β³-aminoacyles soit compris entre 4 et 8, caractérisée en ce que les peptides cycliques sont choisis parmi les hexapeptides et les octapeptides.

Au sens de la présente invention on entend par protéogénique tous les aminoacides naturels ou synthétiques qui entrent dans la constitution des protéines ou des polypeptides, en particulier: l'acide aspartique (Asp ou D), l'asparagine (Asn ou N), la thréonine (Thr ou T), la sérine (Ser ou S), l'acide glutamique (Glu ou E), la glutamine (Gln ou Q), la glycine (Gly ou G), l'alanine (Ala ou A), la cystéine (Cys ou C), la valine (Val ou V), la méthionine (Met ou M), l'isoleucine (Ile ou I), la leucine (Leu ou L), la tyrosine (Tyr ou Y), la phénylalanine (Phe ou F), l'histidine (His ou H), la lysine (Lys ou K), le tryptophane (Trp ou W), la proline (Pro ou P) et l'arginine (Arg ou R).

Dans un mode de réalisation avantageux de l'invention, les peptides cycliques sont choisis parmi les hexapeptides et les octapeptides.

Avantageusement, les résidus L-α-aminoacyles sont choisis dans le groupe comprenant les résidus des aminoacides suivants : arginine (Arg ou R), leucine (Leu ou L), lysine (Lys ou K), phénylalanine (Phe ou F), sérine (Ser ou S), thréonine (Thr ou T) et tryptophane (Tryp ou W) et dans les résidus aza-β³-aminoacyles de formule (A), RI <4a> dans le groupe comprenant des résidus d'aminoacides naturels ou des résidus d'aminoacides non naturels.

Dans un autre mode avantageux de réalisation de l'invention les résidus d'aminoacides naturels entrant dans la structure des résidus d'aza-β³-aminoacyle sont choisis dans le groupe comprenant les résidus d'arginine, de leucine, de lysine, de phénylalanine, de sérine, de thréonine et de tryptophane et les résidus d'aminoacides non naturels sont choisis dans le groupe comprenant les résidus de 1-naphtylalanine [(1)Nal], de 2-naphtylalanine [(2)Nal], de 4-phényl-phénylalanine (4Bip), de diphénylalanine (Dip), de 9-anthracénylalanine [(9)Ath], de 4-pyridylalanine [(4)Pal], de 3-pyridylalanine [(3)Pal], de 2-pyridylalanine [(2)Pal], fluorophénylalanine (4-Fpa), de dodecylalanine (Amy), de Nonylalanine (Non), de Octylalanine (Oct), de cyclohexylalanine (Cha), de 4-fluoro-1-naphtylalanine (4-F-l-Nal), d'homosérine (Hse), d'homo γ-hydroxythréonine, et de Norleucine (Nle).

Dans un mode particulièrement avantageux de l'invention, les peptides cycliques présentent la séquence (Ia) :

c[aza-β³aa_{1'}-aa₁-aza-β³aa_{2'}-aa₂-aza-β³aa_{3'}-aa₃-(aza-β³aa_{4'})m₄ -(aa₄)n₄] (la)

dans laquelle,
chacun des aa₁, aa₂, aa₃, et aa₄ représente indépendamment l'un de l'autre un résidu L-α-aminoacyle,
chacun des aza-β³aa_{1'}, aza-β³aa_{2'}, aza-β³aa_{3'} et aza-β³aa_{4'} représente indépendamment l'un de l'autre un aza-β³-aminoacide, répondant à la formule (A), et
m₄ et n₄ représentent chacun simultanément 0 ou 1,
ou la séquence (Ib) :

c[aa₁- aza-β³aa_{1'}-aa₂-aza-β³aa₂'-aa₃-aza-aza-β³aa_{3'}-(aa₄)n₄-(aza-β³aa_{4'})m₄] (Ib)

dans laquelle,
chacun des aa₁, aa₂, aa₃, et aa₄ représente indépendamment l'un de l'autre un résidu L-α-aminoacyle,
chacun des aza-β³aa_{1'} aza-β³aa_{2'}, aza-β³aa_{3'} et aza-β³aa_{4'} représente indépendamment l'un de l'autre un aza-β³-aminoacide, répondant à la formule (A), et
m₄ et n₄ représentent chacun simultanément 0 ou 1.
ou la séquence (Ic) :
c(aza β³ aa_{1'}-aza β³ aa_{2'}-aza β³ aa_{3'}-aa₁-aza β³ aa_{4'}-aa₂) (Ic)

   dans laquelle,
chacun des aa₁ et aa₂, représente indépendamment l'un de l'autre un résidu L-α-aminoacyle,
chacun des aza-β³aa_{1'}, aza-β³aa_{2'}, aza-β³aa_{3'} et aza-β³aa_{4'} représente indépendamment l'un de l'autre un aza-β³-aminoacide, répondant à la formule (A) ou la séquence (Id) :

   c(aza β³ aa_{1'}-aza β³ aa_{2'}-aa₁-aza β³ aa_{1'}-aa₂) (Id)

   dans laquelle,
chacun des aa₁ et aa₂, représente indépendamment l'un de l'autre un résidu L-α-aminoacyle,
chacun des aza-β³aa_{1'} et aza-β³aa_{2'} représente indépendamment l'un de l'autre un aza-β³-aminoacide, répondant à la formule (A).

Dans un mode particulier de réalisation de l'invention, les peptides cycliques sont choisis dans le groupe comprenant les séquences suivantes :
SEQ ID NO 1 : c[-Leu-*aza*β³Lys-Lys-*aza*β³(1)Nal-Leu-*aza*β³(1)Nal-] (NafNaf),
SEQ ID NO 2 : c[-Lys-*aza*β³Lys-Leu-*aza*β³(1)Nal-Leu-*aza*β³(1)Nal-] (NalNal),
SEQ ID NO 3 : c[-Lys-*aza*β³Lys-Trp-*aza*β³Leu-Trp-*aza*β³Leu-] (TrypTryp),
SEQ ID NO 4 : c[-Lys-*aza*β³Lys-Lys-*aza*β³Leu-Trp-*aza*β³Leu-] (3KW),
SEQ ID NO 5 : c[-Trp-*aza*β³Lys-Lys-*aza*β³Lys-Trp-*aza*β³Leu-] (3K2),
SEQ ID NO 6 : c[-Lys-*aza*β³Lys-Lys-*aza*β³Lys-Trp-*aza*β³(1)Nal-] (4KW),
SEQ ID NO 7 : c[-Ser-*aza*β³(1)Nal-Phe-*aza*β³Lys-Thr-*aza*β³Lys-Ser-*aza*β³Lys-] (SNalF),
SEQ ID NO 8 : c[-Leu-*aza*β³Arg-Arg-*aza*β³(1)Nal-Leu-*aza*β³(1)Nal-] (RNaf),
SEQ ID NO 9 : c[-Leu-*aza*β³Lys-Lys-*aza*β³(1)Nal-*aza*β³Leu-*aza*β³(1)Nal-] (Nafaza),
SEQ ID NO 10 : c[-Phe-*aza*β³Lys-Lys-*aza*β³(1)Nal-Phe-*aza*β³(1)Nal-] (PheNal),
SEQ ID NO 13 :c[-Leu-*aza*β³Lys-Lys-*aza*β³(1)-Nal-Leu-*aza*β³(4F-1)Nal-] (4FluolNaf),
SEQ ID NO 14 : c[-Lys-*aza*β³Lys-Trp-*aza*β³Oct-Trp-*aza*β³Oct-] (OctOct),
SEQ ID NO 15 : c[-Lys-*aza*β³Lys-Cha-*aza*β³Amy-Trp-*aza*β³(1)Nal-] (Chado),
SEQ ID NO 16 : c[-Arg-*aza*β³Lys-Cha-*aza*β³(4)Fpa-Trp-*aza*β³Cha-] (FluoKil),
SEQ ID NO 17 : c[-Leu-*aza*β³Lys-Lys-*aza*β³(2)Nal-Leu-*aza*β³(2)Nal-] (Naf2Naf2),
SEQ ID NO 18: c[-Phe-*aza*β³Lys-Arg-*aza*β³(2)Nal-Phe-*aza*β³(2)Nal-] (PheNar2),
SEQ ID NO 19 : c[-Leu-*aza*β³Lys-Lys-*aza*β³(1)Nal-Leu-*aza*β³(4)Fluo-(1)Nal-] (4FluoNaf),
SEQ ID NO 20 : c[-Leu-*aza*β³Lys-Lys-*aza*β³Bip-Leu-*aza*β³Bip-] (BipBip),

Les peptides cycliques selon l'invention présentent l'avantage d'exclure totalement la présence de D-α-aminoacides, aminoacides non naturels de coût élevé.

Conformément à l'invention, les peptides cycliques peuvent être préparés par toute technique connue de l'homme du métier à partir de produits disponibles dans le commerce ou préparés selon des techniques décrites dans la littérature. Ils peuvent notamment être préparés selon la technique décrite par Busnel O, Bi L, Dali H, Cheguillaume A, Chevance S, Bondon A, Muller S, Baudy-Floch M., J Org Chem. 2005 Dec 23;70(26):10701-8.

Les peptides cycliques selon l'invention se sont révélés actifs sur des souches bactériennes, notamment sur des bactéries à Gram négatif de Groupe 2 (pathogènes) : *Escherichia coli, Salmonella thyphimurium*, *Pseudomonas aeruginosa, Klebsiella pneumoniae* et des bactéries à Gram positif: *Bacillus megaterium (groupe1)* et *Streptococcus aureus (groupe 2).*

En revanche ces pseudopeptides cycliques ne provoquent pas de lyse des cellules sanguines et sont peu ou pas cytotoxiques à des concentrations où ils inhibent pourtant la prolifération bactérienne de bactéries pathogènes.

Les peptides cycliques selon l'invention peuvent être utilisés à titre de médicaments ou pour la désinfection et l'antisepsie (désinfection de surface...) chez l'homme ou chez l'animal, notamment chez les mammifères et les oiseaux.

Avantageusement ils peuvent être utilisés comme antiinfectieux ou antimicrobiens notamment comme antibactériens, antibiotiques, antiparasitaires, antiviraux, antimycotiques ou antifongiques.

L'invention a également pour objet des compositions pharmaceutiques comprenant au moins un peptide cyclique selon l'invention.

Dans ce cadre ils peuvent être utilisés en association avec tout excipient pharmaceutiquement acceptable et sous toute forme pharmaceutiquement acceptable comme par exemple les comprimés, les gélules, les capsules, les savons et les lotions.

Dans un mode de réalisation avantageux de l'invention, les peptides cycliques peuvent être associés avec au moins un autre antünfectieux notamment choisi dans le groupe comprenant la pénicilline, la vancomycine, l'érythromycine ou avec d'autres agents thérapeutiques.

Conformément à l'invention, les peptides cycliques peuvent donc être utilisés pour traiter toutes les maladies infectieuses, notamment les infections nosocomiales, la tuberculose, la septicémie, la pneumonie et les maladies respiratoires.

L'invention a donc également pour objet l'utilisation des peptides cycliques selon l'invention pour la préparation d'un médicament destiné au traitement des maladies infectieuses choisies dans le groupe comprenant notamment les infections nosocomiales, la tuberculose, la septicémie, la pneumonie et les maladies respiratoires.

L'invention a également pour but un méthode de traitement des infections chez l'homme ou chez l'animal comprenant la mise en contact des cellules microbiennes avec au moins un peptide cyclique selon l'invention en une quantité suffisante pour induire la mort des cellules microbiennes sans induire la mort des cellules dudit humain ou dudit animal.

Ils peuvent également être utilisés comme désinfectants en cosmétologie, pour la désinfection de surfaces ou dans le domaine agroalimentaire.

Les exemples 1 et 2 et les figures 1 à 6 qui suivent illustrent l'invention.
La figure 1 illustre la synthèse du peptide NafNaf conformément à l'exemple 1.
La figure 2A représente les concentrations minimales inhibitrices de la prolifération bactérienne (MIC) de peptides selon l'invention sur différentes souches ; la première valeur correspond à la concentration la plus faible inhibant l'activité bactérienne et la deuxième valeur correspond à la dernière concentration à laquelle on observe encore une croissance bactérienne, la valeur de MIC étant comprise entre ces deux valeurs ; la figure 2B représente les concentrations minimales bactéricides (MBC) du peptide NafNaf sur différentes souches.
La figure 3 représente l'activité antibactérienne du peptide NafNaf à des doses croissantes sur *Staphylococcus aureus* mesurée selon l'exemple 2. La colonne « PBB + Bact » correspond au témoin où les bactéries ne sont pas mises en présence de pseudopeptides, la colonne « PBB » correspond au témoin sans bactéries et la colonne « Ampi 1 µg/ml» correspond à l'ampicilline prise comme témoin d'une activité antibactérienne.
La figure 4 représente l'activité antibactérienne du peptide NafNaf à des doses croissantes sur *Salmonella typhimurium* mesurée selon l'exemple 2. La colonne « PBB + Bact » correspond au témoin où les bactéries ne sont pas mises en présence de pseudopeptides, la colonne « PBB » correspond au témoin sans bactéries et la colonne « Ampi 1 µg/ml» correspond à l'ampicilline prise comme témoin d'une activité antibactérienne.
La figure 5 représente la courbe d'activité hémolytique du pseudopeptide cyclique NafNaf mesurée selon la méthode décrite dans l'exemple 3.
La figure 6 illustre la cytotoxicité du pseudopeptide NafNaf à différentes concentrations sur des cellules normales et des cellules cancéreuses.
La figure 7 représente les concentrations minimales inhibitrices de la prolifération bactérienne (MIC) après 48 heures dans le milieu de culture riche TSB (Tryptic Soy Broth) sur différentes souches pour des peptides selon l'invention. L'activité antibactérienne est mesurée selon l'exemple 5. ND : Activité antibactérienne non détectée dans la gamme de concentrations testée ; + Bactéries Gram positives ; - bactéries Gram négatives
La figure 8 représente la courbe d'activité hémolytique de peptides selon l'invention mesurée selon la méthode décrite dans l'exemple 6.

### Exemple 1 : Préparation du pseudo-peptide cyclique NafNaf

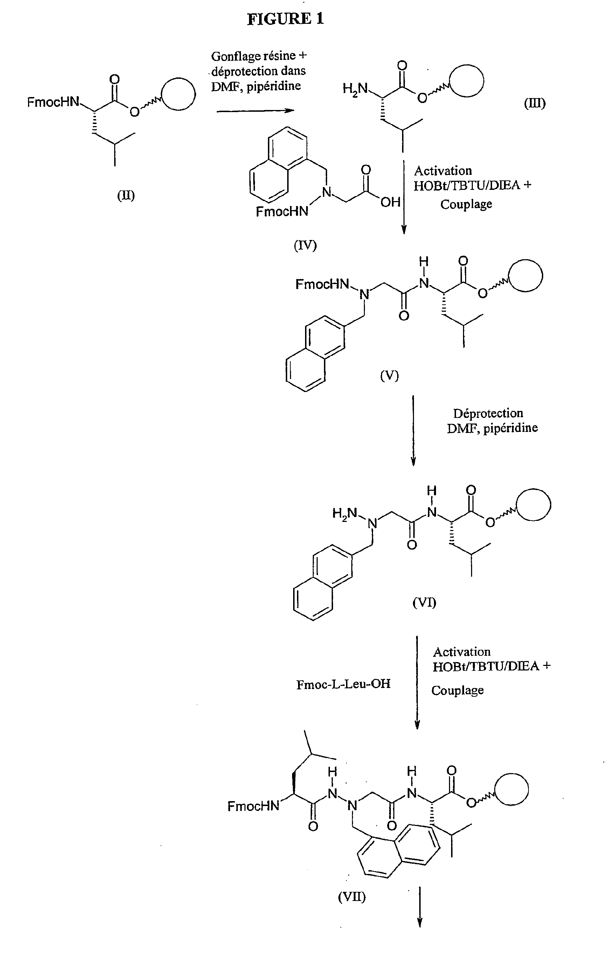

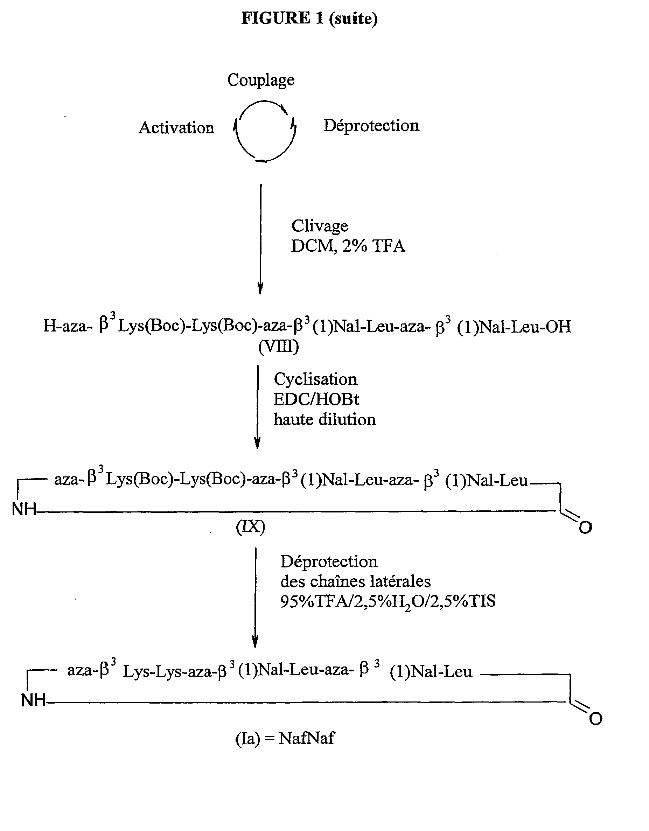

Elle est donnée dans la figure 1. Les chiffres romains utilisés dans le paragraphe qui suit se réfèrent à ladite figure.

Une résine à base de chlorure de 2-chlorotrityle chargée à raison de 1 mmole/g avec de la L-leucine dont la fonction amine est protégée par un groupement Fmoc (9-Fluorénylméthyloxycarbonyl) (II) est mise à gonfler par flux continu de diméthylformamide (DMF) pendant 5 minutes. Le groupe amine de la leucine fixée sur la résine est déprotégé par passage sur la résine d'une solution de pipéridine à 20 % dans du DMF pour donner (III) ; le Fmoc-aza-β³-(1)Nal-OH de formule (IV) est activé par un mélange N-Hydroxybenzotriazole/O-benzotriazole-N,N,N',N'-tetramethyl-uronium-hexafluorophosphate/N-Ethyldiisopropyl amine (HOBt/TBTU/DIEA) dans des proportions (1/1/2) en solution dans le DMF puis couplé à la résine déprotégée (III) pendant 2 heures en flux continu et on obtient (V). On réalise ensuite une déprotection du groupe amine par élimination du groupe Fmoc par passage sur la résine d'une solution de pipéridine à 20 % dans du DMF pour donner (VI) puis on réalise un nouveau couplage dans les conditions décrites précédemment pour obtenir (VII). On recommence le cycle activation-couplage-déprotection autant de fois que nécessaire puis on sépare le peptide final de son support par clivage dans du dichlorométhane (DCM) contenant 2% d'acide trifluoracétique (TFA). On obtient le composé de formule (VIII) que l'on cyclise dans un milieu 1-éthyl-3-(3-diméthylaminopropyl)carbodiimide (EDC)/HOBt à haute dilution (4.10⁻⁴ M)) en présence de 4 équivalents d'agents de couplage HOBt, EDC et DIEA ; cette cyclisation dure deux jours. On obtient le composé (IX) que l'on traite par un mélange TFA/H₂O/TIS (95/2,5/2,5) pour déprotéger les chaînes latérales et obtenir le composé (la) ou NafNaf.

### Exemple 2 : Mesure de l'activité antibactérienne en milieu pauvre PB

### 2.1. Mode opératoire

Les tests ont été réalisés sur des souches bactériennes à Gram négatif de Groupe 2 (pathogènes) : *Escherichia coli, Salmonella thyphimurium, Pseudomonas aeruginosa, Klebsiella pneumonie* et sur des souches à Gram positif : *Bacillus megaterium* (*groupe 1)* et *Streptococcus aureus (groupe 2).*

Les bactéries sont incubées dans un milieu pauvre **PB** pour les bactéries : *Escherichia coli, Pseudomonas aeruginosa, Klebsiella pneumoniae, Bacillus megaterium* et dans un milieu pauvre enrichi en extrait de boeuf **PBB** pour deux autres bactéries: *Streptococcus aureus* et *Salmonella thyphimurium* avec des concentrations croissantes de peptides ou analogues allant de 1 à 320µg/ml. Ce test est réalisé en microplaques de 96 puits. Après une incubation de 18 heures à 30°C ou 37°C, une lecture de la densité optique des microplaques à 595 nm est réalisée afin d'évaluer la croissance bactérienne et de déterminer la concentration minimale d'inhibition (MIC) pour chaque molécule testée. Chaque mesure est réalisée en triplicat.

Par ailleurs la concentration minimale bactéricide (MBC) est déterminée en étalant le contenu des puits où il y a absence de croissance bactérienne sur des boites contenant des milieux riches (*Luria broth* ou (LB) ou *Brain Heart Infusion* (BHi)). La MBC correspond à la concentration à laquelle aucune colonie bactérienne n'est détectée après une incubation de 24 heures à 30 ou 37°C.

### 2.2. Résultats

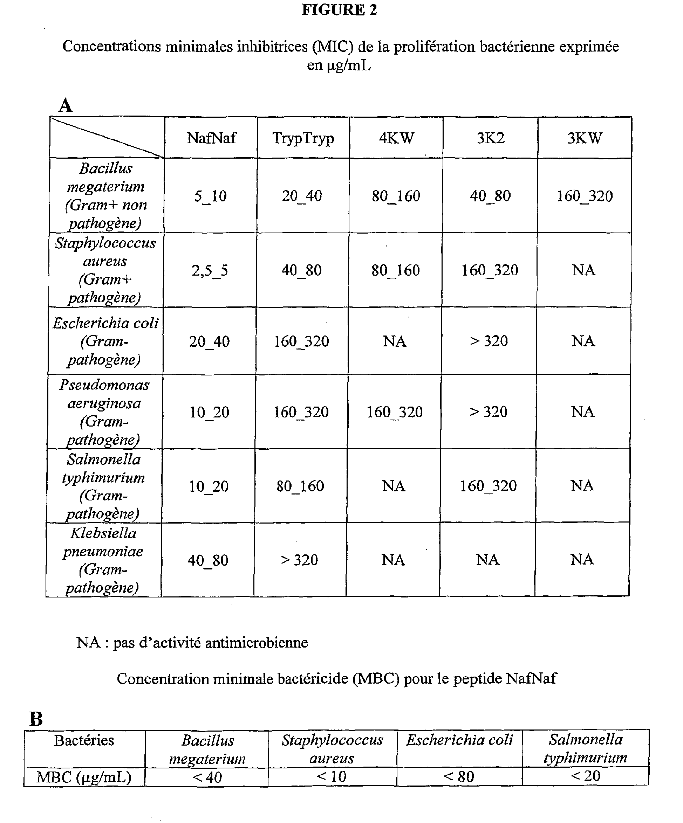

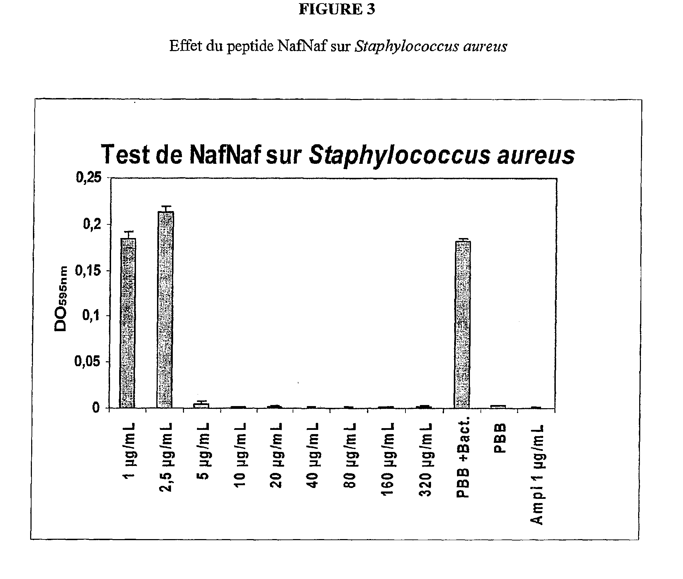

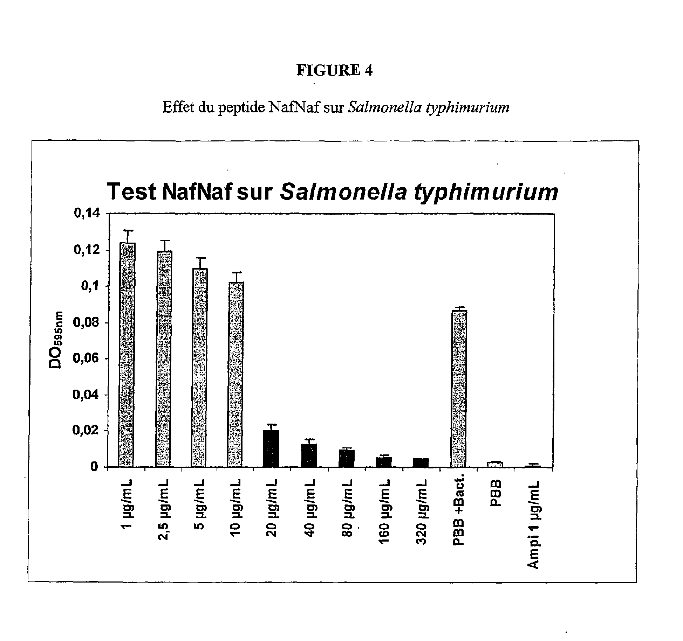

Ils sont donnés dans le tableau de la figure 2 et dans les figures 3 et 4.

Les cinq séquences testées se sont révélées actives bien que seules deux séquences (NafNaf et TrypTryp) présentent des concentrations inhibitrices de la prolifération bactérienne (MIC) inférieures à 100µg/mL (figure 2).

La séquence NafNaf possède des MIC inférieures à 100µg/mL contre toutes les bactéries testées (figure 2).

Ce peptide est très actif sur le staphylocoque doré (*Streptococcus aureus*) responsable de nombreuses maladies nosocomiales avec une MIC inférieure à 5 µg/mL et une MBC inférieure à 10 µg/mL (figure 2).

Il est également actif sur des bactéries pathogènes gram négatives relativement résistantes comme *K*. *pneumoniae* responsables d'infections respiratoires et intestinales (figure 2). Son activité antimicrobienne est de l'ordre des meilleurs peptides cycliques alternant acides aminés D et L avec des MIC d'une dizaine de µg/mL pour les bactéries de gram positif et comprises entre 15 et 80 µg/mL pour les gram négatives.

Le pseudopeptide NafNaf est actif à des concentrations d'intérêt sur l'ensemble des souches étudiées notamment sur des bactéries pathogènes telles que *Streptococcus aureus* et *Salmonelle thyphimurium* où il est actif pour des concentrations inférieures à 20 µg/mL. (figures 3 et 4).

L'Ampicilline (Ampi) choisie comme référence est active à parti de 1 µg/ml pour *Streptococcus aureus* et *Salmonella thyphimurium*. Cette concentration peut être variable selon la souche bactérienne étudiée.

### Exemple 3: Mesure de l'activité hémolytique du peptide NafNaf sur des érythrocytes de lapin

### 3.1. Mode opératoire

L'activité hémolytique des pseudopeptides est testée sur des cellules sanguines de lapin. Les érythrocytes frais de lapin sont lavés trois fois avec un tampon phosphate salin [tampon PBS (pH 7,4)] par centrifugation de 5 min à 900 g et le culot final est suspendu dans du PBS. Les solutions du pseudopeptide NafNaf diluées dans du PBS (90 µL) sont disposées dans une plaque 96 puits. A ces solutions, on ajoute 90 µL de solution d'érythrocytes de lapin à 2% (v/v) pour aboutir à un volume final de 180 µL et une concentration de 1% (v/v) de cellules sanguines. Les suspensions obtenues sont incubées 1 heure à 37°C. Les échantillons sont ensuite centrifugés à 500 g pendant 5 min. 100 µL de surnageant de chaque échantillon sont transférés dans une nouvelle plaque 96 puits. La libération de l'hémoglobine est quantifiée en mesurant l'absorbance à 415 nm du surnageant grâce à un lecteur de microplaques. Les contrôles correspondant à l'absence d'hémolyse (blanc) et à une hémolyse de 100% consistent, respectivement, à ajouté 90 µL de tampon PBS et 90 µL d'une solution à 2% de Triton X-100 aux 90 µL de solution de cellules sanguines à 2%. Chaque mesure est réalisée en triplicat.

### 3.2. Résultats

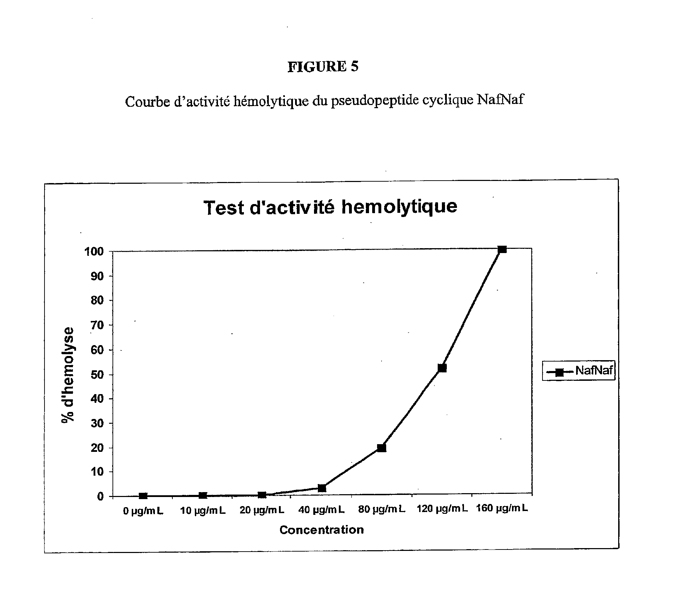

Ils sont donnés dans la figure 5.

La concentration du peptide NafNaf à laquelle on a 50% d'hémolyse est de 120µg/mL.

Cette concentration est plus élevée que celle du peptide cyclique de structure proche alternant des acides aminés L et D et répondant à la formule suivante :

[-(D)Lys-(L)Lys-(D)Leu-(L)Trp-(D)Leu-(L)Trp-]

qui est hémolytique à 80µg/mL.

Ainsi NafNAf ne présente-t-il pas d'activité hémolytique aux concentrations auxquelles il est bactéricide, notamment il n'a pas d'action sur les érythrocytes à 20 µg/mL, concentration à laquelle il est bactéricide pour *Staphylococcus aureus* et *Salmonella typhimurium*.

### Exemple 4 : Evaluation de la cytotoxicité in vitro

### 4.1. Mode opératoire

Les effets des différents pseudopeptides cycliques sur la croissance cellulaire sont évalués dans des plaques 96 puits stériles. Les cellules sont ensemencées à 5.10⁵ cellules par mL de milieu de culture (100µL par puits). Les différents pseudopeptides (10µL par puits) dilués dans une solution saline sont ajoutés au moment de l'ensemencement à la concentration appropriée (de 1µg/mL à 1 mg/mL). Les incubations sont réalisées à 37°C sous atmosphère humidifiée contenant 5% de CO₂ pendant 24 ou 48 heures.

Après l'exposition aux composés, la croissance cellulaire est évaluée en mesurant la formation du formazan à partir du 3-(4,5-dimethylthiazol-2yl)-2,5-diphenyltetrazolium (MTT). L'absorbance à 540 nm est mesurée par un lecteur de microplaques Titertek Multiscan MCC/340. Chaque mesure est réalisée en triplicat.

### 4.2. Résultats

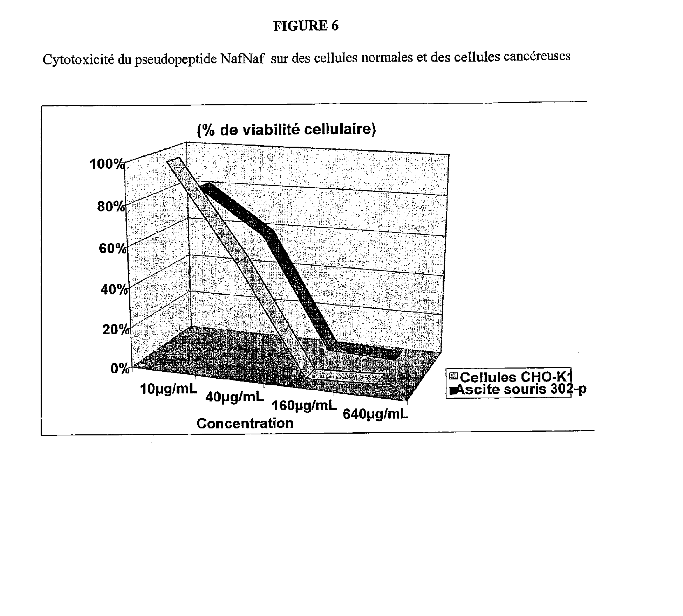

Ils sont donnés dans la figure 6.

La cytotoxicité du pseudopeptide NafNaf a été mesurée sur des cellules de hamster (CHO-K1) et sur une lignés de cellules de souris (Ascite 302-sp).

Ce pseudopeptide n'a aucun effet cytotoxique sur les cellules de hamster à 10µg/mL, concentration bactéricide pour des souches bactériennes comme *S. aureus* et la dose létale pour 50% des cellules (LD₅₀) est d'environ 40µg/mL.

Ces résultats obtenus avec les cellules de hamster non cancéreuses sont confirmés par les résultats obtenus sur la lignée de cellules de souris Ascite 302-sp qui est une lignée cellulaire dérivée de cellules cancéreuses. La LD50 est légèrement plus faible mais on a quand même un léger effet sur la viabilité cellulaire à 10µg/mL.

### Exemple 5 : Mesure de l'activité bactérienne en milieu riche

### 5.1. Mode opératoire

Les tests ont été réalisés sur des souches bactériennes à Gram négatif : *Escherichia coli ATCC 25922, Salmonella enterica ATCC 13076, Pseudomonas aeruginosa ATCC 27853, Klebsiella oxytoca CIP 7932, Entreobacter aerogenes ATCC 13048 et Aeromonas caviae ATCC15468 et sur des souches à Gram positif : Staphylococcus aureus ATCC 25923, Enterococcus faecalis CIP 186, Streptococcus equinus ATCC 5623, Listeria monocytogenes SOR 100, Bacillus megaterium ATCC 10778, Lactococcus gaviae ATCC 43921, Micrococcus luteus ATCC 10240*.

Les bactéries (10⁵ UFC/mL) sont incubées dans un milieu riche TSB (Tryptic Soy Broth) avec des concentrations croissantes de cyclopseudopeptides (0.78, 3.12, 6.25, 12.5, 25, 50, 100 µM). Ce test est réalisé en microplaques de 96 puits. Après une incubation de 48 heures à 20°C ou 37°C, une lecture de la densité optique des microplaques à 595 nm est réalisée afin d'évaluer la croissance bactérienne et de déterminer la concentration minimale d'inhibition (MIC) pour chaque molécule testée. Chaque mesure est réalisée en triplicat.

### 5.2. Résultats

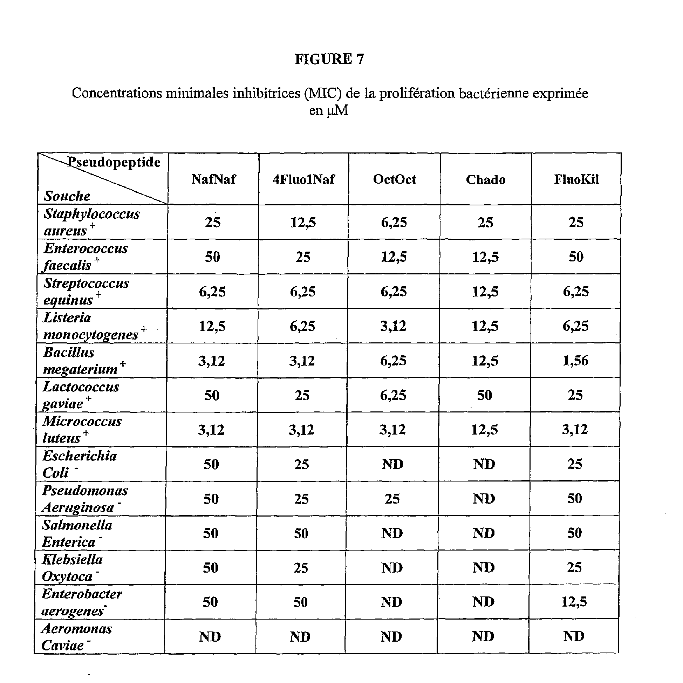

Ils sont donnés dans le tableau de la figure 7.

Les cinq séquences testées se sont révélées actives.

Les cinq cyclopseudopeptides sont capables d'inhiber la croissance de toutes les bactéries à Gram positif testées (bactéries de *S.aureus* à *M. luteus* dans le tableau de la figure7) pour des valeurs de concentrations inférieures ou égales à 50 µM (MIC ≤ 50µM).

En revanche les cyclopseudopeptides apparaissent moins actifs sur les bactéries à Gram négatif (bactéries de *E.coli* à *A.caviae* tableau de la figure7) et l'un d'entre-eux (Chado) ne présente d'ailleurs aucune activité sur les bactéries à Gram négatif aux concentrations testées. Les séquences OctOct et Chado sont composées de résidus aza-ß³-aminoacides portant des chaînes alkyles (C8 ou C12) comme chaînes latérales. Cette modification chimique semble ainsi induire une activité antibactérienne préférentiellement dirigée contre les bactéries à Gram négatif.

*A. caviae* est résistante à tous les cyclopseudopeptides aux concentrations testées et la prolifération des cinq autres souches bactériennes à Gram négatif est inhibée par les cyclopseudopeptides NafNaf, 4 FluoNaf et Fluokill pour des valeurs de MIC comprises entre 12,5 et 50 µM.

Certaines valeurs de MIC étant inférieures à 10 µM sur des bactéries potentiellement pathogènes pour l'homme comme *L. monocytogenes* et *S. equinus*, une application thérapeutique chez l'homme est envisageable.

### Exemple 6: Mesure de l'activité hémolytique de différents peptides selon l'invention sur des érythrocytes de mouton.

### 6.1. Mode opératoire

L'activité hémolytique des pseudopeptides est testée sur des cellules sanguines de mouton. Les érythrocytes de mouton sont lavés trois fois avec un tampon phosphate salin [tampon PBS (pH 7,4)] par centrifugation de 5 min à 900 g et le culot final est suspendu dans du PBS. Pour la concentration de la solution érythrocytaire, on se place à une concentration en globules rouges correspondant à une DO₅₄₀ₙₘ pour le puit contrôle 100% d'hémolyse en présence de Triton X-100 de 0,8. Les solutions des différents pseudopeptides diluées dans de l'eau (100 µL) sont disposées dans une plaque 96 puits. A ces solutions, on ajoute 50 µL de solution tampon PBS 2X puis 50 µL de solution d'érythrocytes de mouton pour aboutir à un volume final de 200 µL. Les suspensions obtenues sont incubées 1 heure à 37°C. Les échantillons sont ensuite centrifugés à 500 g pendant 5 min. 130 µL de surnageant de chaque échantillon sont transférés dans une nouvelle plaque 96 puits. La libération de l'hémoglobine est quantifiée en mesurant l'absorbance à 540 nm du surnageant grâce à un lecteur de microplaques. Les contrôles correspondant à l'absence d'hémolyse (blanc) et à une hémolyse de 100% consistent, respectivement, à ajouté 100 µL de tampon PBS et 100 µL d'une solution à 2% de Triton X-100 aux puits contenant 100 µL de solution érythrocytaire saline (50 µL de PBS 2X et 50 µL de solution de cellules sanguines). Chaque mesure est réalisée en triplicat.

### 6.2. Résultats

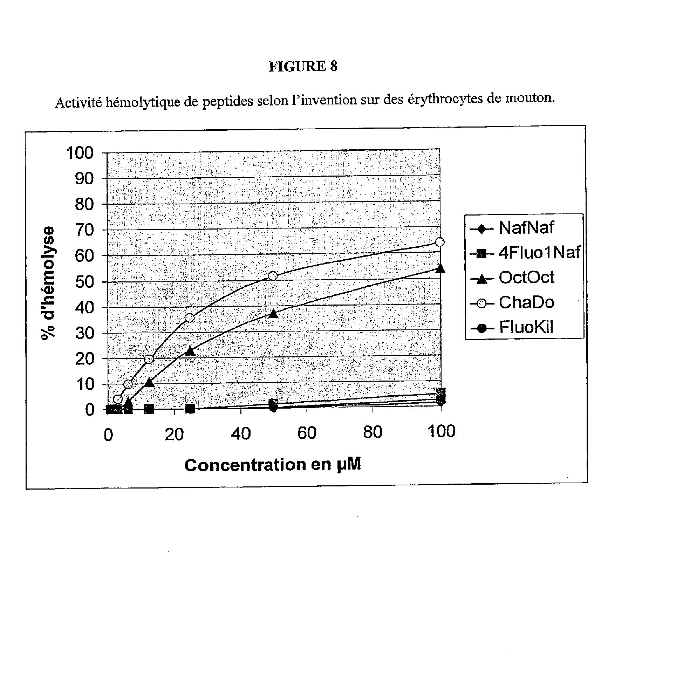

Ils sont donnés dans la figure 8.

Les composés NafNaf, 4-Fluo-1-Naf, et FluoKil ne présentent pas d'activité hémolytique significative aux doses testées.

OctOct et ChaDo présentent une activité hémolytique maximale de respectivement 52 % et 61 % à 100 µM. Néanmoins, à 12,5 µM, concentration à laquelle ils inhibent la croissance de plusieurs bactéries à Gram positif, leurs activités hémolytiques sont moindres de l'ordre de 10% pour OctOct et de 20% pour Chado.

## Revendications

1. Peptides cycliques présentant une alternance aléatoire de résidus L-α-aminoacyles et de résidus aza-β³-aminoacyles répondant à la formule (A) qui suit : 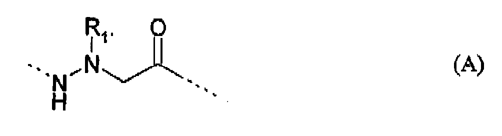 dans laquelle R1 représente une chaîne latérale choisie dans le groupe comprenant les chaînes protéogéniques et non protéogéniques
à condition que deux résidus L-α-aminoacyles soient séparés par au moins un résidu aza-β³-aminoacyle et que le nombre total des résidus L-α-aminoacyles et des résidus axa-β³-aminoacyles soit compris entre 4 et 8, **caractérisée en ce que** les peptides cycliques sont choisis parmi les hexapeptides et les octapeptides.

2. Peptides cycliques selon l'une quelconque des revendications précédentes **caractérisés en ce que** les résidus L-α-aminoacyles sont choisis dans le groupe comprenant les résidus des aminoacides suivants : arginine, leucine, lysine, phénylalanine, sérine, thréonine et tryptophane.

3. Peptides cycliques selon l'une quelconque des revendications précédentes **caractérisés en ce que** dans les résidus aza-β³-aminoacyles de formule (A), R1 représente une chaîne latérale choisie dans le groupe comprenant des résidus d'aminoacides naturels ou des résidus d'aminoacides non naturels.

4. Peptides cycliques selon la revendication 3 **caractérisés en ce que** les résidus d'aminoacides naturels sont choisis dans le groupe comprenant les résidus d'arginine, de leucine, de lysine, de phénylalanine, de sérine, de thréonine et de tryptophane.

5. Peptides cycliques selon la revendication 3 **caractérisés en ce que** les résidus d'aminoacides non naturels sont choisis dans le groupe comprenant les résidus de 1-naphtylalanine [(1)Nal], de 2-naphtylalanine [(2)Nal], de 4-phényl-phénylalanine (4Bip), de diphénylalanine (Dip), de 9-anthracénylalanine [(9)Ath], de 4-pyridylalanine [(4)Pal], de 3-pyridylalanine [(3)Pal], de 2-pyridylalanine [(2)Pal], fluorophénylalanine (4-Fpa), de dodecylalanine (Amy), de Nonylalanine (Non), de Octylalanine (Oct), de cyclohehyalanine (Cha), de 4-fluoro-1-naphtylalanine (4-F-1-Nal), d'homosérine (Hse), d'homo γ-hydroxythréonine, et de Norleucine (Nle).

6. Peptides cycliques selon la revendication 1 présentant la séquence (Ia) :
c[aza-β³aa_{1'}-aa₁-aza-β³aa_{2'}-aa₂-aza-β³aa_{3'}-aa3-( aza-β³aa_{4'})m₄-(aa₄)n₄] (Ia)
dans laquelle,
chacun des aa₁, aa₂, aa₃, et aa₄ représente indépendamment l'un de l'autre un résidu L-α-aminoacyle,
chacun des aza-β³aa_{1'}, aza-β³aa_{2'}, aza-β³aa_{3'} et aza-β³aa_{4'} représente indépendamment l'un de l'autre un aza-β³-aminoacide, répondant à la formule (A), et
m₄ et n₄ représentent chacun simultanément 0 ou 1.

7. Peptides cycliques selon la revendication 1 présentant la séquence (Ib) :
c[aa₁- aza-β³aa_{1'}-aa₂-aza-β³aa_{2'}-aa₃-aza-β³aa_{3'}-(aa₄)n₄- (aza-β³aa_{4'})m₄] (Ib)
dans laquelle,
chacun des aa₁, aa₂, aa₃, et aa₄ représente indépendamment l'un de l'autre un résidu L-α-aminoacyle,
chacun des aza-β³aa_{1'}, aza-β³aa_{2'}, aza-β³aa_{3'} et aza-β³aa_{4'} représente indépendamment l'un de l'autre un aza-β³-aminoacide, répondant à la formule (A), et
m₄ et n₄ représentent chacun simultanément 0 ou 1.

8. Peptides cycliques selon la revendication 1 présentant la séquence (Ic) :
c(aza β³ aa_{1'}-aza β³ aa_{2'}-aza β³ aa_{3'}-aa₁-aza β³ aa_{4'}-aa₂) (Ic)
dans laquelle,
chacun des aa₁ et aa₂ représente indépendamment l'un de l'autre un résidu L-α-aminoacyle,
chacun des aza-β³aa_{1'}, aza-β³aa_{2'}, aza-β³aa_{3'} et aza-β³aa_{4'} représente indépendamment l'un de l'autre un aza-β³-aminoacide, répondant à la formule (A).

9. Peptides cycliques selon la revendication 1 présentant la séquence (Id) :
c(aza β³ aa_{1'}-aza β³ aa_{2'}-aa₁-aza β³ aa_{1'}-aa₂) (Id)
dans laquelle,
chacun des aa₁ et aa₂ représente indépendamment l'un de l'autre un résidu L-α-aminoacyle,
chacun des aza-β³aa_{1'} et aza-β³aa_{2'} représente indépendamment l'un de l'autre un aza-β³-aminoacide, répondant à la formule (A).

10. Peptides cycliques selon l'une quelconque des revendications 6 à 9 **caractérisés en ce qu'**ils sont choisis dans le groupe comprenant les séquences suivantes :
SEQ ID NO 1: c[Leu-*aza*β³Lys-Lys-*aza*β³(1)Nal-Leu-*azaβ*³(1)Nal-] (NafNaf),
SEQ ID NO 2 : c[Lys-*aza*β*³*Lys-Leu-*aza*β*³*(1)Nal-Leu-*aza*β*³*(1)Nal-] (NalNal),
SEQ ID NO 3 : c[Lys-*aza*β*³*Lys-Trp-*aza*β*³*Leu-Trp-*aza*β*³*Leu-] (TrypTryp),
SEQ ID NO 4: c[Lys-*aza*β*³*Lys-Lys-*aza*β*³*Leu-Trp-*aza*β*³*Leu-] (3KW),
SEQ ID NO 5 : c[Trp-*aza*β*³*Lys-Lys-*aza*β*³*Lys-Trp-*aza*β*³*Leu-] (3K2),
SEQ ID NO 6: c[Lys-*aza*β*³*Lys-Lys-*aza*β*³*Lys-Trp-*aza*β*³*(1)Nal-] (4KW),
SEQ ID NO7: c[Ser-*aza*β*³*(1)Nal-Phe-*aza*β*³*Lys-Thr-*aza*β*³*Lys-Ser-*aza*β*³*Lys-] (SNalF),
SEQ ID NO 8; c[Leu-*aza*β*³*Arg-Arg-*aza*β*³*(1)Nal-Leu-*aza*β*³*(1)Nal-] (RNaf),
SEQ ID NO 9 : c[Leu-*aza*β*³*Lys-Lys-*aza*β*³*(1)Nal-*aza*β*³*Leu-*aza*β*³*(1)Nal-] (Nafaza),
SEQ ID NO 10 : c[Phe-*aza*β*³*Lys-Lys-*aza*β*³*(1)Nal-Phe-*aza*β*³*(1)Nal-] (PheNal),
SEQ ID NO 13 :c[-Leu-*aza*β*³*Lys-Lys-*aza*β*³*(1)-Nal-Leu-*aza*β*³*(4F-1)Nal-] (4Fluo1Naf),
SEQ ID NO 14 : c[-Lys-*aza*β*³*Lys-Trp-*aza*β*³*Oct-Trp-*aza*β*³*Oct-] (OctOct),
SEQ ID NO 15: c[-Lys-*aza*β*³*Lys-Cha-*aza*β*³*Amy-Trp-*aza*β*³*(1)Nal-] (Chado),
SEQ ID NO 16 : c[-Arg-*aza*β*³*Lys-Cha-*aza*β*³*(4)Fpa-Trp-*aza*β*³*Cha-] (FluoKil).
SEQ ID NO 17 c[-Leu-*aza*β*³*Lys-Lys-*aza*β*³*(2)Nal-Leu-*aza*β*³*(2)Nal-] (Naf2Naf2),
SEQ ID NO 18 : c[-Phe-*aza*β*³*Lys-Arg-*aza*β*³*(2)Nal-phe-*aza*β*³*(2)Nal-] (PheNar2),
SEQ ID NO 19 : c[-Leu-*aza*β*³*Lys-Lys-*aza*β*³*(1)Nal-Leu-*aza*β*³*(4)Fluo-(1)Nal-] (4FluoNaf),
SEQ ID NO 20 : c[-Leu-*aza*β*³*Lys-Lys-*aza*β*³*Bip-Leu-*aza*β*³*Bip-1 (BipBip),

11. Peptides cycliques selon l'une quelconque des revendications précédentes à titre de médicaments.

12. Peptides cycliques selon la revendication 11 **caractérisés en ce que** le médicament est un antiinfectieux ou un désinfectant.

13. Composition pharmaceutique comprenant au moins un peptide cyclique selon l'une des revendications 1 à 10 associés à tout excipient pharmaceutiquement acceptable.

14. Utilisation d'au moins une peptide selon l'une quelconque des revendications 1 à 10 ou d'une composition pharmaceutique selon la revendication 13 pour la préparation d'un médicament destiné au traitement des maladies infectieuses.

## Patentansprüche

1. Zyklische Peptide, die eine zufällige Abfolge von L-α-Aminoacylresten und Aza-β³-Aminoacylresten mit der folgenden Formel (A) aufweisen: 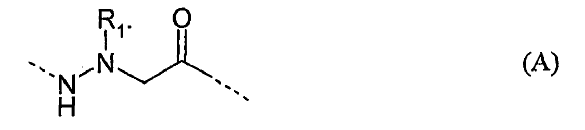 worin R1 für eine Seitenkette steht, ausgewählt aus der Gruppe umfassend proteogene und nicht proteogene Ketten,
unter der Voraussetzung, dass zwei L-α-Aminoacylreste durch mindestens einen Aza-β³-Aminoacylrest getrennt sind und dass die Gesamtanzahl der L-α-Aminoacylreste und Aza-β³-Aminoacylreste im Bereich zwischen 4 und 8 liegt, **dadurch gekennzeichnet, dass** die zyklischen Peptide ausgewählt sind aus Hexapeptiden und Octapeptiden.

2. Zyklische Peptide nach einem der vorhergehenden Ansprüche, **dadurch gekennzeichnet, dass** die L-α-Aminoacylreste ausgewählt sind aus der Gruppe, umfassend die folgenden Aminosäurereste: Arginin, Leucin, Lysin, Phenylalanin, Serin, Threonin und Tryptophan.

3. Zyklische Peptide nach einem der vorhergehenden Ansprüche, **dadurch gekennzeichnet, dass** in den Aza-β³-Aminoacylresten der Formel (A) R1 für eine Seitenkette steht, ausgewählt aus der Gruppe, umfassend natürliche Aminosäurereste oder nicht natürliche Aminosäurereste.

4. Zyklische Peptide nach Anspruch 3, **dadurch gekennzeichnet, dass** die natürlichen Aminosäurereste ausgewählt sind aus der Gruppe, umfassend Arginin-, Leucin-, Lysin-, Phenylalanin-, Serin-, Threonin- und Tryptophanreste.

5. Zyklische Peptide nach Anspruch 3, **dadurch gekennzeichnet, dass** die nicht natürlichen Aminosäurereste ausgewählt sind aus der Gruppe, umfassend 1-Naphtylalanin- [(1)Nal], 2-Naphtylalanin-[(2)Nal], 4-Phenylphenylalanin- (4Bip), Diphenylalanin- (Dip), 9-Anthracenylalanin- [(9)Ath], 4-Pyridylalanin- [(4)Pal], 3-Pyridylalanin- [(3)Pal], 2-Pyridylalanin- [(2)Pal], Fluorphenylalanin- (4-Fpa), Dodecylalanin- (Amy), Nonylalanin- (Non), Octylalanin-(Oct), Cyclohexylalanin- (Cha), 4-Fluor-1-naphtylalanin- (4-F-1-Nal), Homoserin- (Hse) und Homo-γ-hydroxythreonin- und Norleucinreste (Nie).

6. Zyklische Peptide nach Anspruch 1, die die Sequenz (Ia) aufweisen:
c[aza-β³aa_{1'}-aa₁-aza-β³aa_{2'}-aa₂-aza-β³aa_{3'}-aa₃- (aza-β³aa_{4'})m₄- (aa₄)n₄] (Ia)
worin:
aa₁, aa₂, aa₃ und aa₄ jeweils unabhängig voneinander für einen L-α-Aminoacylrest stehen,
aza-β³aa_{1'}, aza-β³aa_{2'}, aza-β³_{aa3'} und aza-β³aa_{4'} jeweils unabhängig voneinander für eine Aza-β³-Aminosäure mit der Formel (A) stehen, und
m₄ und n₄ jeweils gleichzeitig für 0 oder 1 stehen.

7. Zyklische Peptide nach Anspruch 1, die die Sequenz (Ib) aufweisen:
c [aa₁-aza-β³aa_{1'} -aa₂-aza- β³aa_{2'} -aa₃-aza-β³aa_{3'}- (aa₄) n₄-(aza-β³aa_{4'})m₄] (Ib)
worin:
aa₁, aa₂, aa₃ und aa₄ jeweils unabhängig voneinander für einen L-α-Aminoacylrest stehen,
aza-β³aa_{1'}, aza-β³aa_{2'}, aza-β³aa_{3'} und aza-β³aa_{4'} jeweils unabhängig voneinander für eine Aza-β³-Aminosäure mit der Formel (A) stehen, und
m₄ und n₄ jeweils gleichzeitig für 0 oder 1 stehen.

8. Zyklische Peptide nach Anspruch 1, die die Sequenz (Ic) aufweisen:
c(aza β³ aa_{1'}-aza β³ aa_{2'}-aza β³ aa_{3'}-aa₁-aza β³ aa_{4'}-aa₂) (Ic)
worin:
aa₁ und aa₂ jeweils unabhängig voneinander für einen L-α-Aminoacylrest stehen,
aza-β³aa_{1'}, aza-β³aa_{2'}, aza-β³aa_{3'} und aza-β³aa_{4'} jeweils unabhängig voneinander für eine Aza-β³-Aminosäure mit der Formel (A) stehen.

9. Zyklische Peptide nach Anspruch 1, die die Sequenz (Id) aufweisen:
c(aza β³ aa_{1'}-aza β³ aa_{2'}-aa₁-aza β³ aa_{1'}-aa₂) (Id)
worin:
aa₁ und aa₂ jeweils unabhängig voneinander für einen L-α-Aminoacylrest stehen,
aza-β³aa₁, und aza-β³aa_{2'} jeweils unabhängig voneinander für eine Aza-β³-Aminosäure mit der Formel (A) stehen.

10. Zyklische Peptide nach einem der Ansprüche 6 bis 9, **dadurch gekennzeichnet, dass** sie ausgewählt sind aus der Gruppe, umfassend die folgenden Sequenzen:
SEQ ID NO 1: c[Leu-*aza*β*³*Lys-Lys-*aza*β*³*(1)Nal-Leu-*aza*β*³*(1)Nal-] (NafNaf),
SEQ ID NO 2: c[Lys-*aza*β*³*Lys-Leu-*aza*β*³*(1)Nal-Leu-*aza*β*³*(1)Nal-] (NalNal),
SEQ ID NO 3: c[Lys-*aza*β*³*Lys-Trp-*aza*β*³*Leu-Trp-*aza*β*³*Leu-] (TrypTryp),
SEQ ID NO 4: c[Lys-*aza*β*³*Lys-Lys-*aza*β*³*Leu-Trp-*aza*β*³*Leu-] (3KW),
SEQ ID NO 5: c[Trp-*aza*β*³*Lys-Lys-*aza*β*³*Lys-Trp-*aza*β*³*Leu-] (3K2),
SEQ ID NO 6: c[Lys-*aza*β*³*Lys-Lys-*aza*β*³*Lys-Trp-*aza*β*³*(1)Nal-] (4KW),
SEQ ID NO 7: c[Ser-*aza*β*³*(1)Nal-Phe-*aza*β*³*Lys-Thr-*aza*β*³*Lys-Ser-*aza*β*³*Lys-] (SNalF),
SEQ ID NO 8: c[Leu-*aza*β*³*Arg-Arg-*aza*β*³*(1)Nal-Leu-*aza*β*³*(1)Nal-] (RNaf),
SEQ ID NO 9: c[Leu-*aza*β*³*Lys-Lys-*aza*β*³*(1)Nal-*aza*β*³*Leu-*aza*β*³*(1)Nal-] (Nafaza),
SEQ ID NO 10: c[Phe-*aza*β*³*Lys-Lys-*aza*β*³*(1)Nal-Phe-*aza*β*³*(1)Nal-] (PheNal),
SEQ ID NO 13: c[Leu-*aza*β*³*Lys-Lys-*aza*β*³*(1)Nal-Leu-*aza*β*³*(4F-1)Nal-] (4Fluo1Naf),
SEQ ID NO 14: c[-Lys-*aza*β*³*Lys-Trp-*aza*β*³*Oct-Trp-*aza*β*³*Oct-] (OctOct),
SEQ ID NO 15: c[-Lys-*aza*β*³*Lys-Cha-*aza*β*³*Amy-Trp-*aza*β*³*(1)Nal-] (Chado),
SEQ ID NO 16: c[-Arg-*aza*β*³*Lys-Cha-*aza*β*³*(4)Fpa-Trp-*aza*β*³*Cha-] (FluoKil),
SEQ ID NO 17: c[-Leu-*aza*β*³*Lys-Lys-*aza*β*³*(2)Nal-Leu-*aza*β*³*(2)Nal-] (Naf2Naf2),
SEQ ID NO 18: c[-Phe-*aza*β*³*Lys-Arg-*aza*β*³*(2)Nal-Phe-*aza*β*³*(2)Nal-] (PheNar2),
SEQ ID NO 19: c[-Leu-*aza*β*³*Lys-Lys-*aza*β*³*(1)Nal-Leu-*aza*β*³(*4)Fluo-(1)Nal-] (4FluoNaf),
SEQ ID NO 20: c[-Leu-*aza*β*³*Lys-Lys-*aza*β*³*Bip-Leu-*aza*β*³*Bip-] (BipBip).

11. Zyklische Peptide nach einem der vorhergehenden Ansprüche als Medikamente.

12. Zyklische Peptide nach Anspruch 11, **dadurch gekennzeichnet, dass** das Medikament ein Antiinfektivum oder ein Desinfektivum ist.

13. Pharmazeutische Zusammensetzung, umfassend mindestens ein zyklisches Peptid nach einem der Ansprüche 1 bis 10 in Verbindung mit jedem pharmazeutisch verträglichen Exzipienten.

14. Verwendung mindestens eines Peptids nach einem der Ansprüche 1 bis 10 oder einer pharmazeutischen Zusammensetzung nach Anspruch 13 zur Herstellung eines Medikaments, das zur Behandlung von Infektionskrankheiten bestimmt ist.

## Claims

1. Cyclic peptides having a random alternation of L-α-aminoacyl residues and of aza-β³-aminoacyl residues corresponding to formula (A) below: 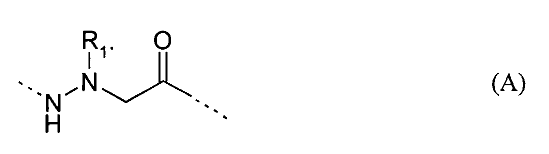 in which R1 represents a side chain selected from the group comprising proteogenic and non-proteogenic chains
provided that two L-α-aminoacyl residues are separated by at least one aza-β³-aminoacyl residue and that the total number of the L-α-aminoacyl residues and the aa-β³-aminoacyl residues is comprised between 4 and 8, **characterized in that** the cyclic peptides are selected from the hexapeptides and the octapeptides.

2. Cyclic peptides according to any one of the previous claims **characterized in that** the L-α-aminoacyl residues are selected from the group comprising the residues of the following amino acids: arginine, leucine, lysine, phenylalanine, serine, threonine and tryptophan.

3. Cyclic peptides according to any one of the previous claims **characterized in that** in the aza-β³-aminoacyl residues of formula (A), R1 represents a side chain selected from the group comprising natural amino acid residues or non-natural amino acid residues.

4. Cyclic peptides according to claim 3 **characterized in that** the natural amino acid residues are selected from the group comprising arginine, leucine, lysine, phenylalanine, serine, threonine and tryptophan residues.

5. Cyclic peptides according to claim 3 **characterized in that** the non-natural amino acid residues are selected from the group comprising 1-naphthylalanine [(1)Nal], 2-naphthylalanine [(2)Nal], 4-phenyl-phenylalanine (4Bip), diphenylalanine (Dip), 9-anthracenylalanine [(9)Ath], 4-pyridylalanine [(4)Pal], 3-pyridylalanine [(3)Pal], 2-pyridylalanine [(2)Pal], fluorophenylalanine (4-Fpa), dodecylalanine (Amy), nonylalanine (Non), octylalanine (Oct), cyclohexylalanine (Cha), 4-fluoro-1-naphthylalanine (4-F-1-Nal), homoserine (Hse), homo γ-hydroxythreonine, and norleucine (Nle) residues.

6. Cyclic peptides according to claim 1 having the sequence (Ia):
c[aza-β³aa_{1'}-aa₁-aza-β³aa_{2'}-aa₂-aza-β³aa_{3'}-aa₃-(aza-β³aa_{4'})m₄-(aa₄)n₄] (Ia)
in which,
each of aa₁, aa₂, aa₃, and aa₄ represents independently from each other an L-α-aminoacyl residue,
each of aza-β³ aa_{1'}, aza-β³aa_{2'}, aza-β³aa_{3'} and aza-β³aa_{4'} represents independently from each other an aza-β³-amino acid, corresponding to formula (A), and
m₄ and n₄ each simultaneously represent 0 or 1.

7. Cyclic peptides according to claim 1 having the sequence (Ib):
c[aa₁- aza-β³aa₁-aa₂-aza-β³aa_{2'}-aa₃-aza-β³aa_{3'}-(aa₄)n₄- (aza-β³aa_{4'})m₄] (Ib)
in which
each of the aa₁, aa₂, aa₃, and aa₄ represents independently from each other an L-α-aminoacyl residue,
each of aza-β³aa_{1'}, aza-β³aa_{2'}, aza-β³aa_{3'} and aza-β³aa_{4'} represents independently from each other an aza-β³-amino acid, corresponding to formula (A), and
m₄ and n₄ each simultaneously represent 0 or 1.

8. Cyclic peptides according to claim 1 having the sequence (Ic):
c(aza β³ aa_{1'}-aza β³ aa_{2'}-aza β³ aa_{3'}-aa₁-aza β³ aa_{4'}-aa₂) (Ic)
in which
each one of aa₁ and aa₂ represents independently of each other an L-α-aminoacyl residue,
each one of aza-β³aa_{1'}, aza-β³aa_{2'}, aza-β³aa_{3'} and aza-β³aa_{4'} represents independently from each other an aza-β³-amino acid, corresponding to formula (A).

9. Cyclic peptides according to claim 1 having the sequence (Id):
c(aza β³ aa_{1'}-aza β³ aa_{2'}-aa₁-aza β³ aa_{1'}-aa₂) (Id)
in which
each one of aa₁ and aa₂ represents independently from each other an L-α-aminoacyl residue,
each one of aza-β³aa_{1'} and aza-β³aa_{2'} represents independently from each other an aza-β³-amino acid, corresponding to formula (A).

10. Cyclic peptides according to any one of claims 6 to 9 **characterized in that** they are selected from the group comprising the following sequences:
SEQ ID NO 1: c[Leu-*aza*β³Lys-Lys-*aza*β³(1)Nal-Leu-*aza*β³(1)Nal-] (NafNaf),
SEQ ID NO 2: c[Lys-*aza*β*³*Lys-Leu-*aza*β*³*(1)Nal-Leu-*aza*β*³*(1)Nal-] (NalNal),
SEQ ID NO 3: c[Lys-*aza*β*³*Lys-Trp-*aza*β*³*Leu-Trp-*aza*β*³*Leu-] (TrypTryp),
SEQ ID NO 4: c[Lys-*aza*β*³*Lys-Lys-*aza*β*³*Leu-Trp-*aza*β*³*Leu-] (3KW),
SEQ ID NO 5: c[Trp-*aza*β*³*Lys-Lys-*aza*β*³*Lys-Trp-*aza*β*³*Leu-] (3K2),
SEQ ID NO 6: c[Lys*-aza*β*³*Lys-Lys-*aza*β*³*Lys-Trp-*aza*β*³*(1)Nal-] (4KW),
SEQ ID NO7: c[Ser-*aza*β*³*(1)Nal-Phe-*aza*β*³*Lys-Thr-*aza*β*³*Lys-Ser-*aza*β*³*Lys-] (SNalF),
SEQ ID NO 8: c[Leu-*aza*β*³*Arg-Arg-*aza*β*³*(1)Nal-Leu-*aza*β*³*(1)Nal-] (RNaf),
SEQ ID NO 9: c[Leu-*aza*β*³*Lys-Lys-*aza*β*³*(1)Nal-*aza*β*³*Leu-*aza*β*³*(1)Nal-] (Nafaza),
SEQ ID NO 10: c[Phe-*aza*β*³*Lys-Lys-*aza*β³*(*1)Nal-Phe-*aza*β*³*(1)Nal-] (PheNal),
SEQ ID NO 13:c[-Leu-*aza*β*³*Lys*-*Lys-*aza*β*³*(1)-Nal-Leu-*aza*β*³*(4F-1)Nal-] (4Fluo1Naf),
SEQ ID NO 14: c[-Lys-*aza*β*³*Lys-Trp-*aza*β*³*Oct-Trp-*aza*β*³*Oct-] (OctOct),
SEQ ID NO 15: c[-Lys-*aza*β*³*Lys-Cha-*aza*β*³*Amy-Trp-*aza*β*³*(1)Nal-] (Chado),
SEQ ID NO 16: c[-Arg-*aza*β*³*Lys-Cha-*aza*β*³*(4)Fpa-Trp-*aza*β*³*Cha-](FluoKil).
SEQ ID NO 17: c[-Leu-*aza*β*³*Lys-Lys-*aza*β*³*(2)Nal-Leu-*aza*β*³*(2)Nal-] (Naf2Naf2),
SEQ ID NO 18: c[-Phe-*aza*β*³*Lys-Arg-*aza*β*³*(2)Nal-Phe-*aza*β*³*(2)Nal-] (PheNar2),
SEQ ID NO 19: c[-Leu-*aza*β*³*Lys*-*Lys-*aza*β*³*(1)Nal-Leu-*aza*β*³*(4)Fluo-(1)Nal-] (4FluoNaf),
SEQ ID NO 20: c[-Leu-*aza*β*³*Lys-Lys-*aza*β*³*Bip-Leu-*aza*β*³*Bip-] (BipBip),

11. Cyclic peptides according to any one of the previous claims as a medicament.

12. Cyclic peptides according to claim 11 **characterized in that** the medicament is an anti-infective agent or a disinfectant.

13. Pharmaceutical composition comprising at least one cyclic peptide according to one of claims 1 to 10 combined with any pharmaceutically acceptable excipient.

14. Use of at least one peptide according to any one of claims 1 to 10 or of a pharmaceutical composition according to claim 13 for preparing a medicament intended for the treatment of infectious diseases.
